# EUROPEAN PATENT APPLICATION

(11) **EP 1 849 482 A1**
(43) Date of publication of application: **31.10.2007**
(21) Application number: 06008502.4
(22) Date of filing: 25.04.2006
(51) Int. Cl.: A61K 47/48, A61K 49/18

(54) **Multimodally altered cells as a form for administering active substances and as diagnostic particles**

(71) Applicant: Capsulution Nanoscience AG, 12489 Berlin (DE); Charité-Universitätsmedizin Berlin, 10117 Berlin (DE)
(72) Inventor: Voigt, Andreas, 12621 Berlin (DE); Bäumler, Hans, 13187 Berlin (DE)
(74) Representative: Leidescher, Thomas

(57) **Abstract**

The present application demonstrates that both active substances and diagnostically effective substances can be introduced into biological cells, in particular blood cells, simultaneously. These multimodally altered cells can be found once again, in vivo and in vitro, using modern imaging methods. In addition to the diagnostic and active compound-carrier principles, they can serve as a form for administering the active substances in an accurately targeted manner.

## Description

### Background to the invention

The spatial and chronological resolution of imaging diagnostic methods has made great progress in recent years. Tomographic methods such as computer tomography (CT) and magnetic resonance tomography (MRT), as well as positron emission tomography (PET) are at the forefront of these methods. These methods can be used to detect and locate disease foci in the body with a high degree of accuracy. However, pharmacological active compounds are generally still being administered systemically and not in an accurately targeted manner. One of the reasons for this is that the diagnostic principles and the principles of administering the active compounds are based on completely independent methods and different substances. [Bildgebende Systeme für die medizinische Diagnostik [Imaging systems for medical diagnosis], Heinz Morneburg, Wiley-VCH Verlag 1995; CT, EBT, MRT und Angiographie [CT, EBT, MRT and angiography], Roland C. Bittner, and others Urban & Fischer 2003; Drug Delivery Systems (Pharmacology and Toxicology: Basic and Clinical Aspects), Vasant V. Ranade, Mannfred A. Hollinger CRC Press 2003].

There is a wealth of investigations into the possibility of enclosing active substances in biological cells, e.g. erythrocytes [Red Blood Cells as Carriers for Drugs, J.R. DeLoach and U. Sprandel (editors), Bibliotheca Haematologica No. 51, S. Karger, Basle, 1985; Red Blood Cells as Carriers for Drugs, C. Ropars, M. Chassaigne and C. Nicolau (editors), Advances in the Biosciences, Volume 67, Pergamon Press, Oxford, New York, 1987; Drug, enzyme and peptide delivery using erythrocytes as carriers, C. Gutiérrez Millán et al., Journal of Controlled Release, 95/1, 2004, 27-49; DE 2656317; US 4289756; WO 92/08804]. In this connection, the uptake of active compound can be configured in such a way that a high proportion of the cells survive. This thereby enables the cells to be returned to the biological system. The active substances which have thus far been encapsulated also include those which could nowadays be used for diagnostic purposes.

### Summary of the invention

In the present use, it has been shown that both active compounds and diagnostically active substances can be introduced for instance simultaneously into the cells. Surprisingly, it has been possible to find these multimodally altered or modified cells once again using modern imaging methods. As a result, it is possible to realize the idea underlying the invention, i.e. that of linking the diagnostic and administration principles to each other and demonstrating this. In addition to the diagnostic and active compound-carrier principles, the cells can also be modified for administering the active substances in a manner which is accurately targeted.

According to the invention, altered or modified human or animal cells, in particular human or animal or mammalian cells, comprise at least one substantially water insoluble active substance which is present in the form of particles or nanoparticles. The cells can further comprise at least one diagnostic substance.

The particles or nanoparticles have an average size of less than 10 µm, preferably less than 1 µm, more preferably less than 200 nm and most preferably less than 100 nm, and have a minimum size of at least 5 nm and preferably more than 10 nm. Nanoparticles are preferred which have a size of less than 1 µm.

In particular, the cells are blood cells and, more particularly, erythrocytes. The diagnostic substance can, in particular, be magnetite. It has been found that active substances which are in the form of nanoparticles can be particularly readily introduced into cells without the latter, in particular the cell membrane, being damaged.

In addition, the invention proposes a method for altering human or animal cells, which method involves the steps of:
- loading the cells with at least one substantially water insoluble active substance which is present in the form of nanoparticles, and
- loading the cells with at least one diagnostic substance.

It is advantageous if the cells are loaded simultaneously with at least one active substance and at least one diagnostic substance.

In this specification substantially water insoluble or water insoluble substances and compounds are those which exhibit a very low solubility in water and which typically require additives to be dissolved in water. Examples are for instance hydrophobic substances and compounds which includes many pharmaceutically active substances (drugs) such as anti-inflammatory and antineoplastic drugs and steroids. Therefore, active substances are in particular pharmaceutically active substances (drugs).

A diagnostic substance is a substance which is used as a marker, e.g. contrast agent, for identifying the altered cells (or blood cells) or for manipulating the altered cells, e.g. by magnetic fields if magnetic substances or nanoparticles are used. Other diagnostic substance are fluorescence labelled molecules, macromolecules and/or nanoparticles.

Without wishing to be tied to a theory, it is assumed that water insoluble substances or compounds, when presenting to cells or blood cells in a form of particles or nanoparticles, are not incorporated into the cell membrane. Until now, attempts have been made to incorporate water insoluble or poorly water soluble active substances or hydrophobic compounds into blood cells such as erythrocytes in molecular form. Thereby, the water insoluble active substances or hydrophobic compounds have mainly been incorporated into the cell membrane of the erythrocytes and have thereby disintegrated the cell membrane which caused for instance haemolysis of the erythrocytes. In contrast thereto, the present invention allows an accumulation of comparably large quantities of particles or nanoparticles containing the hydrophobic or water insoluble active substance or compound without incorporating them into the cell membrane. It is assumed that the particles or nanoparticles are to big to be incorporated into the cell membrane. The uptake of particles or nanoparticles might require the formation of temporary pores in the cell membrane which might cause a partial loss of haemoglobin if erythrocytes are used. However, the pores readily seals and no further haemolysis is observed due to the fact that the nanoparticles are not incorporated into the cell membrane.

Therefore, it has been found by the present inventors that a disintegration of the cell membrane after loading can be substantially reduced if the active compounds are presented to the cells or blood cells in particulate or nanoparticulate form. It is assumed that the incorporation of nanoparticles into the cell membrane is substantially less likely than the incorporation of molecules.

A further advantage of the present invention is that the cells can be loaded with a substantially higher amount of the active substance or compound in comparison with prior art methods which merely allow an incorporation of the active compound or substance into the cell membrane. For instance, in prior art methods using liposomes as carriers the concentration of the incorporated active substance or compound in the lipid membrane of the liposomes is rather low in comparison with the present method which allows an accumulation of the nanoparticles in the cell volume.

The surface of the cells or blood cells can be modified to enable specific interaction with or reaction of the immune system of a target organism to which the altered blood cells are applied, macrophages, cancel cells or other cells. Modification of the surface includes, but is not limited to, coating with a plurality of polymer layers such as polyelectrolytes, binding of antibodies and antigens and covalent binding of macromolecules.

The altered cells can be used for diagnostic purposes and evaluation of therapies.

It is preferred to alter the cells by incorporating the active substance or substances which are present in the form of nanoparticles and the diagnostic substance or substances.

A further advantage of the present invention is that the loading of the cells can be carried out in closed systems, i.e. systems or equipment which ensure an sterile handling of the cells so that they can be re-infused subsequently. This can for instance take place in modified equipments, which are otherwise used for manufacturing blood preserves.

Moreover, the loading of the cells can be carried out *in vivo.* This is particularly true for monocytes and granulocytes which selectively phagocytise modified erythrocytes containing the active compound or substance, carry the active compound or substance through the blood-brain barrier and subsequently release it there. The monocytes or granulocytes are not damaged when phagocytising the erythrocytes or other modified cells in contrast to *ex vivo* procedures which substantially stimulate these cells. Therefore, blood cells such as monocytes or granulocytes can be loaded by first accumulating the active substance or compound in erythrocytes or other suitable cells and than presenting the loaded cells to monocytes or granulocytes which will subsequently phagocytise them. Optionally, the surface of the loaded cells can be modified to stimulate or enhance the phagocytosis.

### Exemplary embodiments

### (1) Loading the erythrocytes with magnetite - MRT

Human or animal blood is treated with an anticoagulant. After that, the erythrocytes are separated from the remaining blood constituents by centrifugation. The plasma and the remaining blood constituents are removed. The erythrocytes are washed in physiological saline. The erythrocytes are resuspended in a hypotonic and cooled solution which contains the magnetite particles. This suspension is agitated moderately on a roller-shaker for approx. 1 hour. After the erythrocytes have been incubated in the magnetite solution, the suspension is centrifuged in order to separate the erythrocytes from the suspension solution.

The latter is removed and the erythrocytes are washed in physiological solution at room temperature. The quantity of the magnetite which is present in the erythrocytes can be determined by means of MRT or by means of a chemical iron determination. The magnetite-loaded erythrocytes can be returned to the donor's blood stream if the procedures have been carried out under sterile conditions.

### (2) Labeling with fluorescent dyes (antibodies) - detection using a flow cytometer

Blood cells, such as erythrocytes, are washed as described in exemplary embodiment (1). The erythrocytes are then resuspended in a salt solution which contains polymers which are labeled with fluorescent dyes. These polymers adsorb to the erythrocyte surface. A small quantity of this suspension is then introduced into the measuring channel of a suitable flow cytometer such that each individual cell is detected on the basis of the fluorescence which is excited by the laser radiation. Nonerythrocytic constituents of the suspension are recognized as such because of the lack of the fluorescence signal.

Examplary embodiment (2) can be combined with exemplary embodiment (1).

### (3) Loading the erythrocytes with endoxan - detection by means of phagocytosis

Erythrocytes which have been prepared as described in exemplary embodiment (1) are resuspended in a hypotonic and cooled solution which contains, for example, the active substance endoxan. This suspension is agitated moderately on a roller-shaker for approx. 1 hour. After the erythrocytes have been incubated in this solution, the suspension is centrifuged in order to separate the erythrocytes from the suspension solution. The latter is removed and the erythrocytes are washed in physiological solution at room temperature. The endoxan-loaded erythrocytes are then added to a blood cell culture. The monocytes and granulocytes which are present in this culture recognize the treated erythrocytes and phagocytose them. The quantity of the phagocytosed cells can be determined using a test which enables the living monocytes and granulocytes to be distinguished from those which have died.
Exemplary embodiment (3) can be combined with exemplary embodiments (1) and (2).

In particular, magnetite and active substances, e.g. endoxan, can be enclosed simultaneously in the erythrocytes during the course of a preparation. The preparations in exemplary embodiments (1) and (3) are identical apart from the substance to be enclosed. This self-evidently only applies to substances which are mutually compatible.

### (4) Loading the erythrocytes with Amphotericin B detection by means of phagocytosis

Amphotericin B is mechanically crushed at high pressure in the presence of surfactant (tensides) to a size less than 1 µm and is suspended in water such as to obtain a suspension of nanoparticles (nanosuspension). The average particle size of the nanoparticles are for example about 80 nm. The nanosuspension is cooled at about 4°C.
Erythrocytes which have been prepared as described in exemplary embodiment (1) are resuspended in a hypotonic and cooled solution which contains, for example, the active substance Amphotericin B in the form of nanoparticles. This suspension is agitated moderately on a roller-shaker for approx. 1 hour. After the erythrocytes have been incubated, the suspension is centrifuged in order to separate the erythrocytes from the suspension solution. The latter is removed and the erythrocytes are repeatedly washed in physiological solution at room temperature such that no Amphotericin B is detectable in the supernatant by means of HPLC. For testing the Amphotericin B-loaded erythrocytes are then added to a blood cell culture which contains monocytes and granulocytes in addition to yeast cells. The monocytes and granulocytes recognize the treated erythrocytes and phagocytose them. Upon phagocytosis of the erythrocytes Amphotericin B is released in the monocytes and granulocytes and gradually transported out of the cells such that the yeast cells die. For verification a test is performed using a flow cytometer.

The loading procedure leads for instance to a final concentration of 4pg Amphotericin B per erythrocyte. It was proven that an amount of only 750 Amphotericin B-loaded erythrocytes per ml is sufficient to reach an antifungal effect, representing one millionth of the physiological erythrocyte concentration in human blood (4-6*10⁹ per ml).

The resulting high concentration of Amphotericin B in the cells has been verified by HPLC. Furthermore, using atomic force microscopy and electron microscopy it has been shown that Amphotericin B has mainly been accumulated in the cell but not in the cell membrane.

Exemplary embodiment (4) can be combined with exemplary embodiments (1) and (2).

### (5) Coating erythrocytes with polymers

Native erythrocytes can be coated with suitable polymers simply using adsorption or using the electrostatic interaction of the polymers with the surface of the erythrocytes. The process which dominates depends on the nature of the polymers which are used. Erythrocytes which have been treated as described in exemplary embodiment (1), (3) or (4) are introduced into a salt solution which contains polymers. Since the erythrocytes react sensitively to pH changes and salt concentration changes, with this being able to result in the erythrocytes being destroyed, it is necessary to maintain conditions which do not diverge too strongly from the physiological conditions. The erythrocytes are incubated, for approx. 30 min at room temperature, in the suspension while being agitated moderately. They are then centrifuged and the suspension solution is separated from the erythrocytes. Unbound polymers are removed by carefully washing the erythrocytes several times in suitable salt solutions. This step can be followed by further coating steps using the same procedure. The success of the coating can be established by, for example, determining the change in the zeta potential of the erythrocytes.

Exemplary embodiment (5) can be combined with exemplary embodiments (1), (2), (3) and (4).

For targeting of organs or tissues erythrocytes can be additionally loaded with magnetite nanoparticles as diagnostic substance (average particle size 10nm) to enable magnetically focussing of the erythrocytes to the desired sites. Loading erythrocytes simultaneously with both, e.g. Amphotericin B and magnetite, did neither reduce the Amphotericin B concentration per erythrocyte nor its bioactivity. Optionally, the erythrocytes can be modified with one or several types of antibodies and/or antigens (e.g. Tumor-antibodies). Additionally, magnetite loaded erythrocytes can be visualized by MRI, offering the opportunity for diagnostic monitoring of the therapy.

To achieve effectiveness in CNS fungal infections, Amphotericin B loaded erythrocytes were in incorporated into RES cells. Due to damage of the blood brain barrier (BBB) in infections RES cells cross the BBB in a higher percentage than usual. In-*vitro* phagocytosis assays using Amphotericin B loaded erythrocytes show a significant inhibition of free fungal activity. This effect can be observed in viability assays using flowcytometric analysis and direct cell culture assay.

The advantages of erythrocyte-carrier-system are not limited to Amphotericin B. Any other water insoluble, emulsive drug has potential to be loaded onto erythrocytes thereby exploiting the advantages of the proposed carrier system.

The vitality of the loaded cells, such as erythrocytes, can be extended up to 7 weeks by employing suitable storage solutions and conditions. The *in vivo* survival rate is about several days depending on the loading procedure employed, which has been demonstrated in a rat model using human erythrocytes. However, this model is suboptimal since rats form antibodies against human erythrocytes within a few days. It is therefore very likely that substantially longer survival rates can be achieved if required for special purposes.

Due to their unique qualities erythrocytes can be used as therapeutical drug delivery systems, which cannot just deliver a high dosage of different drugs but also protects them from inactivating effects and minimizes side reactions. The additional diagnostic capacities as MRI contrast media and the possibility to focus on certain tissues make our delivery system one of the most versatile drug carriers.

In principle, the method according to the invention can be used to alter human or animal cells, and in particular human or animal blood cells, by
- loading them with at least one active substance, and
- optionally loading them with at least one diagnostic substance.

After the alteration, the altered cells can be returned to the human or animal body as a cellular form for administering the active substance and as diagnostic cell particles.

Furthermore, the surfaces of the altered cells can also be modified for the purpose of specifically interacting with the biological milieu of the target organism or for the purpose of labeling with fluorescent or optical or magnetic or enzymic or diagnostic labels.

The altered cells, in particular the blood cells, are erythrocytes, platelets, neutrophilic, eosinophilic and basophilic granulocytes, monocytes and lymphocytes, and hematopoietic stem cells and progenitor cells.

In addition, the altered cells can be cells from other organs of the human or animal body or can be genetically transformed cells.

The altered cells can serve as a form for administering the active substances and contain an additional active substances in, for example, the form of
- molecules
- nanoparticles
- colloidal nanoparticles
- micelles
- molecular complexes and molecular aggregates
- emulsions
- liposomes and vesicles
- layer-by-layer particles
- cell constituents

It is also possible for the altered cells, as administration form, to contain the substances in a form which is activated by interaction with endogenous substrates.

It is likewise possible for the altered cells to contain, as administration form, radioactive substances or magnetizable or superparamagnetizable, nanoparticulate substances.

As administration form which contains active substances, the altered cells can release the latter by means, for example, of
- passive processes such as diffusion, permeation or osmosis,
- triggered processes such as a change in permeability, a lysis, heating or a mechanical destruction which bring about a resonance process,
- the action of ultrasound, laser light, magnetic fields, magnetic field pulses, electrical fields or electrical field pulses,
- interaction with other cells, such as phagocytosis or endocytosis, or activation mechanisms.

As diagnostic particles, the altered cells can contain substances which can be detected by means of
- nuclear spin resonance or nuclear spin tomography (MRT)
- X-rays or X-ray computer tomography (CT)
- positron emission tomography (PET)
- fluorescence measurement techniques
- laser techniques
- ultrasonic techniques
- infrared techniques
- other imaging methods

In this connection, the altered cells can, as diagnostic particles, contain diagnosable substances such as
- trivalent cations
- multivalent ions
- luminescent or fluorescent dyes and phosphorescent dyes
- IR absorbers
- magnetic and paramagnetic substances such as magnetite
- polarizable substances
- X-ray contrast media
- ultrasonic contrast media

In addition, the surfaces of the altered cells can be modified by means of
- PEGylation
- the covalent binding-on of peptides, oligonucleotides or oligosaccharides and hybrid forms thereof
- adsorption of monolayers and multilayers such as polyelectrolyte multilayers
- antibodies and antigens
- incorporation of cell membrane constituents and cell vesicles
- change in the lipid matrix
- change in their mechanical properties
- adsorption of ionic and molecular substances
- immobilization of enzymes
- incorporation of receptors
- substances which bring about a change in the permeability properties and substance transfer properties

As a form for administering the active substances, and as diagnostic cell particles, the altered cells can be used
- in isolated organs and organ systems
- in cell cultures in bioreactors, or
- for in-vitro investigations.

In addition, the altered cells should, as the form for administering the active substances and as diagnostic cell particles, be prepared in accordance with the rules and conditions of good laboratory and preparation practice (GLP, GMP) which in each case apply in the fields of pharmacy, medicine, biotechnology and technology.

## Claims

1. Human or animal cell, in particular blood cell, comprising at least one substantially water insoluble active substance which is present in the form of particles or nanoparticles.

2. Human or animal cell as claimed in claim 1 further comprising at least one diagnostic substance.

3. Human or animal cell as claimed in claim 2, wherein the diagnostic substance is a magnetizable or superparamagnetizable nanoparticulate substance, fluorescence labelled molecules, macromolecules and/or nanoparticles.

4. Human or animal cell as claimed in claim 2 or 3, wherein the diagnostic substance is magnetite.

5. Human or animal cell as claimed in one of the preceding claims, wherein the human or animal cell is an erythrocyte, a platelet, a neutrophilic, eosinophilic or basophilic granulocyte, a monocyte or a lymphocyte, or a hematopoietic stem cell or progenitor cell.

6. Human or animal cell as claimed in one of the preceding claims, wherein the cell is surface-modified.

7. Human or animal cell as claimed in claim 6, wherein the surface of the cell is coated with polymers.

8. Human or animal cell as claimed in claim 6 or 7, wherein the surface of the blood cell is coated with polyelectrolyte layers.

9. Method for modifying human or animal cells, in particular human or animal blood cells, comprising the steps of:
loading the cells with at least one water insoluble active substance which is present in the form of particles or nanoparticles.

10. Method as claimed in claim 9, further comprising the step of:
loading the cells with at least one diagnostic substance.

11. Method as claimed in claim 9 or 10, wherein the cells are loaded with the at least one active substance and the at least one diagnostic substance simultaneously.

12. Method as claimed in any of the claims 9 to 11, further comprising the step of:
modifying the surface of the cells.

13. Method as claimed in claim 12, wherein the surface of the cells is modified by coating with polymers.

14. Method as claimed in claim 12 or 13, wherein the surface of the cell is modified by coating with polyelectrolyte layers.

15. Method as claimed in one of claims 9 to 14, wherein the diagnostic substance is a magnetizable or superparamagnetizable nanoparticulate substance.

16. The method as claimed in one of claims 9 to 15, wherein the diagnostic substance is magnetite.

17. The method as claimed in one of claims 9 to 16, wherein the human or animal cell is an erythrocyte, a platelet, a neutrophilic, eosinophilic or basophilic granulocyte, a monocyte or a lymphocyte, or a hematopoietic stem cell or progenitor cell.

18. The use of the modified cell as claimed in one of claims 1 to 8, or of the cells which have been prepared as claimed in one of claims 9 to 17 as the form for administering the active substance and as a diagnostic cell particle
- in isolated organs and organ systems
- in cell cultures
- in bioreactors, or
- for in-vitro investigations.
